Europäisches Patentamt

European Patent Office

Office européen des brevets

(11) Veröffentlichungsnummer: **0 333 642**
A1

## (12) EUROPÄISCHE PATENTANMELDUNG

(21) Anmeldenummer: **89810045.8**

(22) Anmeldetag: **19.01.89**

(51) Int. Cl.⁴: **A 61 F 2/38**

(30) Priorität: **01.03.88 CH 771/88**

(43) Veröffentlichungstag der Anmeldung:
**20.09.89 Patentblatt 89/38**

(84) Benannte Vertragsstaaten: **AT DE FR GB IT**

(71) Anmelder: **GEBRÜDER SULZER
AKTIENGESELLSCHAFT
Zürcherstrasse 9
CH-8401 Winterthur (CH)**

**Protek AG
Stadtbachstrasse 64
CH-3001 Bern (CH)**

(72) Erfinder: **Müller, Maurice E., Prof. Dr.-med.
Melchenbühlweg 9
CH-3006 Bern (CH)**

**Bürgi, Maya
Chaennerwisstrasse 5
CH-8352 Räterschen (CH)**

(54) **Verankerungsplatte für die tibialen Lagerflächen.**

(57) Eine Verankerungsplatte (1) für tibiale Lagerflächen von Femurkondylen ist über Verankerungszapfen (3) lösbar in der Tibia (4) verankert. Die Zapfen (3) sind als Hohlzylinder (5) ausgebildet; die lösbare Verbindung erfolgt durch in der Platte (1) versenkte, von aussen zugängliche Schrauben (10).

Die Ausbildung der Verankerungszapfen (3) als Hohlzylinder (5) verbessert die Haftung des Implantats bei zementfreier Verankerung. Die von aussen lösbare Verbindung zwischen Platte (1) und Zapfen (3) ermöglicht Reoperationen unter weitestgehender Schonung des Knochengewebes der Tibia (4).

Fig. 1

EP 0 333 642 A1

**Beschreibung**

## Verankerungsplatte für die tibialen Lagerflächen

Die Erfindung betrifft eine Verankerungsplatte für die tibialen Lagerflächen von Femurkondylen einer Kniegelenkprothese, wobei im tibialen Plateau der Platte Verankerungszapfen für eine zementfreie Verankerung im Knochen vorgesehen sind.

Die Druckschrift "ICHL-Knieprothesen" der Firma Protek AG, Bern, zeigt Prothesenkonstruktionen, bei denen die tibialen Lagerflächen mit der Verankerungsplatte zu einem einzigen Tibiateil zusammengefasst sind, die im übrigen jedoch die vorstehend erwähnten Merkmale aufweist. In der Praxis hat sich gezeigt, dass die Haftung eines solchen Tibiateils in der Plattform der Tibia bei zementfreier Verankerung unter Umständen ungenügend ist. In Fällen, in denen eine Reoperation erforderlich wird, hat sich darüberhinaus ergeben, dass eine solche nur unter weitgehender Beschädigung der Tibiaplattform möglich ist.

Aufgabe der Erfindung ist es daher, eine Verankerungsplatte zu schaffen, bei der bei gleichzeitiger Verbesserung der Haftung eine zementfreie Verankerung eine Reoperation unter weitgehender Schonung des Knochens möglich ist.

Diese Aufgabe wird dadurch gelöst, dass die Verankerungszapfen als Hohlzylinder ausgebildet sind, in denen Durchbrüche für ein Einwachsen von Knochengewebe vorhanden ist, und dass ferner die Verankerungszapfen derart lösbar mit der Platte verbunden sind, dass die implantierte Platte von aussen von den implantierten Verankerungszapfen abnehmbar ist.

Das Einwachsen von Knochengewebe in die Hohlräume des Zapfens und durch seine Durchbrüche hindurch, verbessert die Fixierung der Zapfen in der Tibia erheblich. Weiterhin ermöglicht die von oben lösbare Verbindung eine Trennung der Platte von dem im Knochen implantierten Hohlzapfen, ohne dass schwerwiegende Beschädigungen der Tibia erforderlich sind. Nach Abnahme der Platte kann dann das in die Zapfen eingewachsene und an sie angewachsene Gewebe mit scharfen, etwa ihrem Innen- bzw. Aussendurchmesser angepassten zylindrischen "Messer" durchtrennt und der Zapfen relativ einfach entfernt werden, wobei im wesentlichen Knochengewebe nur in einem ringförmigen Hohlraum beeinträchtigt und zerstört wird.

Die lösbare Verbindung zwischen Verankerungszapfen und Platte lässt sich auf einfache Weise durch in der Platte versenkte Schrauben verwirklichen, die in ein Gewinde des Zapfenhohlraumes eingreifen und mit denen die Zapfen an der Platte gehalten werden.

Weiterhin lässt sich die zementfreie Verankerung durch Verdichten von spongiosem Knochengewebe verbessern, wenn der Hohlzylinder der Verankerungszapfen am freien Ende eine scharfkantige konische Schneide aufweist.

Im folgenden wird die Erfindung anhand eines Ausführungsbeispiels im Zusammenhang mit der Zeichnung näher erläutert.

Fig. 1 zeigt teilweise im Schnitt eine Ansicht der neuen Verankerungsplatte, eingesetzt in eine Tibia;

Fig. 2 ist eine Aufsicht auf Fig. 1 von oben.

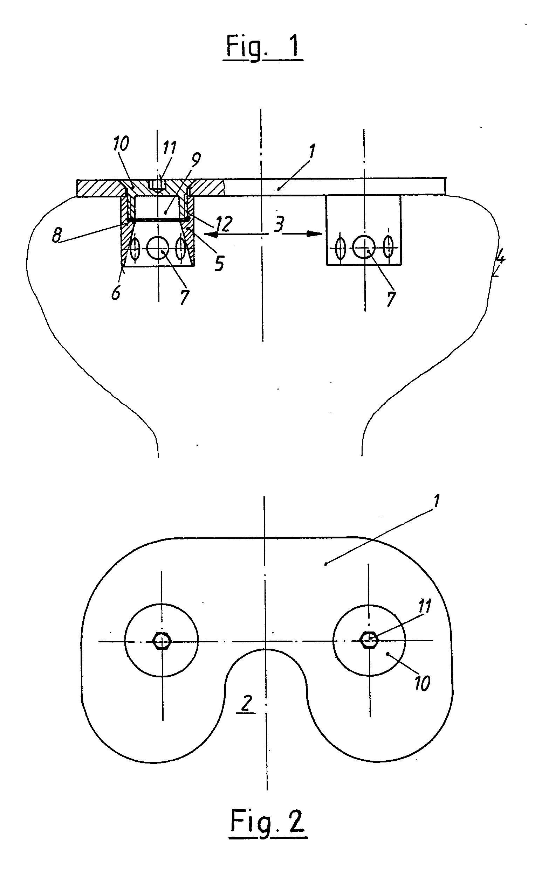

Die Verankerungsplatte 1, die nach dorsal eine Ausnehmung 2 (Fig. 2) für die Kreuzbänder hat, ist mit Verankerungszapfen 3 in der Tibia 4 verankert. Die Zapfen 3 bestehen aus Hohlzylindern 5, deren freies Ende eine relative scharfe Kante 6 bildet, von der aus sich der Hohlraum des Zylinders 5 über etwa 2/3 seiner Höhe konisch verengt. Der Mantel des Zylinders 5 ist mit Durchbrüchen 7 für ein Durchwachsen von Knochengewebe versehen, die auf dem ganzen Umfang des Hohlzylinders 5 gleichmässig verteilt sind.

Die obere Kante der sich konisch verengenden Hohlraumbegrenzung endet in einem Absatz 8, der in einen zylindrischen Hohlraum 9 grösseren Durchmessers überleitet. Dieser Hohlraum 9 ist mit einem Gewinde 12 versehen. In dieses greift ein Gegengewinde einer Schraube 10 ein, die mit ihrem Kopf in der Platte 1 versenkt ist und sich in der Platte 1 abstützt. Mit der Schraube 10, die einen Innensechskant 11 für den Einsatz eines Werkzeugs aufweist, ist der Hohlzylinder 5 über das Gewinde 9 mit der Verankerungsplatte 1 von aussen lösbar verbunden.

**Patentansprüche**

1. Verankerungsplatte für die tibialen Lagerflächen von Femurkondylen einer Kniegelenkprothese, wobei im tibialen Plateau der Platte Verankerungszapfen für eine zementfreie Verankerung im Knochen vorgesehen sind, **dadurch gekennzeichnet, dass** die Verankerungszapfen (3) als Hohlzylinder (5) ausgebildet sind, in denen Durchbrüche (7) für ein Einwachsen von Knochengewebe vorhanden sind, **und dass ferner** die Verankerungszapfen (3) derart lösbar mit der Platte (1) verbunden sind, dass die implantierte Platte (1) von aussen von den implantierten Verankerungszapfen (3) abnehmbar ist.

2. Verankerungsplatte nach Anspruch 1, **dadurch gekennzeichnet, dass** die Verankerungszapfen (3) durch in der Platte (1) versenkte Schrauben (10), die in ein Gewinde (12) des Hohlraumes des Zapfens (3) eingreifen, an der Platte (1) gehalten sind.

3. Verankerungsplatte nach Anspruch 1 oder 2, **dadurch gekennzeichnet, dass** die Hohlzylinder (5) der Verankerungszapfen (3) am freien Ende eine scharfkantige, konische Schneide (6) aufweisen.

Fig. 1

Fig. 2

Europäisches Patentamt

**EUROPÄISCHER RECHERCHENBERICHT**

Nummer der Anmeldung

EP 89 81 0045

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | WO-A-8 502 535 (PROTEK AG) <br> * Figur 16, 6/7 * <br> --- | 1-3 | A 61 F 2/38 |
| X | DE-U-8 713 071 (M. IZADPANAH) <br> * Figur 1 * <br> ----- | 1,2 | |

RECHERCHIERTE SACHGEBIETE (Int. Cl.4)

A 61 F

Der vorliegende Recherchenbericht wurde für alle Patentansprüche erstellt

| Recherchenort | Abschlußdatum der Recherche | Prüfer |
|---|---|---|
| DEN HAAG | 14-06-1989 | ARGENTINI A. |